# EUROPEAN PATENT SPECIFICATION

(11) **EP 3 876 897 B1**
(45) Date of publication and mention of the grant of the patent: **20.12.2023**
(21) Application number: 19795142.9
(22) Date of filing: 22.10.2019
(51) Int. Cl.: A61K 8/02, A61Q 19/00, A61K 8/46

(54) **RED COLORANT FREE OF COCHINEAL RED AND COMPOSITIONS COMPRISING THE SAME**
ROTES FÄRBEMITTEL OHNE COCHENILLE-ROT UND ZUSAMMENSETZUNGEN DAMIT
COLORANT ROUGE EXEMPT DE ROUGE DE COCHENILLE ET COMPOSITIONS LES COMPRENANT

(30) Priority: 09.11.2018 US 201862757824 P
(43) Date of publication of application: 15.09.2021
(60) Divisional application: 22155016.3
(73) Proprietor: UNILEVER GLOBAL IP LIMITED, Wirral Merseyside CH62 4ZD (GB); Unilever IP Holdings B.V., 3013 AL Rotterdam (NL)
(72) Inventor: HUANG, Lei, Trumbull, CT Connecticut 06611 (US); QIU, Qiang, Trumbull, CT Connecticut 06611 (US); ROSA, Jose, Guillermo, Trumbull, CT Connecticut 06611 (US); COURTOIS, Jean-Philippe, Andre, Roger, Wirral Merseyside CH63 3JW (GB)
(74) Representative: James, Helen Sarah
(86) International application number: PCT/EP2019/078678
(87) International publication number: WO 2020/094383

(56) References cited:
- US-A1- 2002 086 039
- US-A1- 2010 172 852
- US-A1- 2016 271 052
- US-A1- 2017 231 889

## Description

### Field of the invention

The present invention is directed to a red colorant composition. Such a red colorant composition mimics the color of cochineal (i.e., carmine) red, and unexpectedly, results in a color composition that is stable, even in the presence of actives conventionally used in cosmetic compositions. The present invention is also directed to an end use composition comprising the red colorant composition of this invention.

### Background of the invention

For many years, pigments, oils and moisturizing agents, like water, have been used in cosmetic compositions. A popular color in cosmetic compositions is red since red is often a color desired for use on a consumer's eye-lids, lips and/or cheeks. In fact, use of red colorants in lipsticks, acne creams, eye shadow, blush, mascara and foundation is common. It is also common to use red colorant in nail polish.

Typically, red colorants that are used in cosmetic compositions can be in the form of lakes, dyes and pigments. A popular red colorant suitable for use in many end use consumer compositions (including food compositions) is cochineal red. Cochineal red, which is essentially crushed and dried insect (*Dactylopius Coccus*), is often used as a preferred colorant. The insect itself produces carmic acid (about 17-24% of a dried insect's weight). When mixed with metal salts (e.g., calcium, magnesium or aluminum) carmine red colorant (cochineal) is obtained. With consumers now demanding to know what precisely exists in the cosmetics they use and foods they consume, it is not always well received when consumers learn that crushed bug is in their cosmetic, food product or even soft drink.

In view of the foregoing, it is of increasing interest to develop a red colorant composition that is stable and mimics the color of cochineal red. This invention, therefore, is directed to a red colorant composition that comprises salts of (sulfonatophenylazo) naphthoate and (phenylazo) naphthalenedisulfonate. The red colorant composition of the present mimics the color of cochinial red, and surprisingly, is stable even in the presence of actives typically used in cosmetic compositions.

### Additional Information

Efforts have been disclosed for making color compositions. In U.S. Patent No. 5,340,569, blush formulations with ultrafine boron nitride and magnesium fatty acid salts are described.

Even other efforts have been disclosed for making color compositions. In U.S. Patent No. 5,108,736, pigmented cosmetic products in the form of a cake, cream, liquid or stick form are described.

Still other efforts have been disclosed for making color compositions. In U.S. Patent No. 8,088,430, compositions for topical application comprising microencapsulated colorants are described.

Additional efforts for making colorants have been disclosed. In WO 2015/044212A1, stable red formulations for foods and beverages are described. US 2016/271052 describes natural red colorants as an alternative to animal-based red colorant.

None of the additional information describes a color composition that mimics the color of cochinial red as described and claimed in the present invention.

### Summary of the invention

In a first aspect, the present invention is directed to a composition according to claim 1.

Skin, as used herein, is meant to include skin on the arms (including underarms) face, feet, neck, chest, hands, legs, buttocks and scalp (including hair). Particle size, as it relates to the first and second components, means the average diameter of the colorants (taken at the widest point) in microns. Particle size may be measured with a commercially available Malvern Mastersizer. The particle sizes of the first and second components of the present invention (when spheres) are typically from 0.5 to 7 microns, including all ranges subsumed therein. If such colorants are plates or platy, they typically have a length and width which are each, independently, 1-50 microns, and a thickness from 75 to 650 nanometers. End use composition (e.g., anhydrous or substantially anhydrous, or water or oil continuous emulsion) is a cosmetic composition for topical application or oral benefits and includes blush, lipstick, lip gloss, foundation, balm, rouge, mascara, powder, nail polish, cream, lotion, serum, gel, primer, highlighter, oral supplement, eye liner, mousse, aerosol, deodorant, antiperspirant, shampoo, conditioner, make-up or personal wash, including bars and liquids. Active or benefit agent, as herein defined, is a component including an oil or water-soluble component that delivers a benefit (including cosmetic benefit) to skin after being topically applied. In one embodiment of this invention, the end use composition is substantially anhydrous.. In another embodiment, the end use composition of this invention is a leave-on skin lotion, cream, liquid or personal wash composition. In still another embodiment of this invention, the end use composition is anhydrous or substantially anhydrous. Anhydrous, as used herein, means free of water and substantially anhydrous (i.e., semi-anhydrous) means from 0.001 to 10% by weight water, and preferably, from 0.01 to 5% by weight water, and most preferably, from 0.1 to 3% by weight water, based on total weight of the end use composition and including all ranges subsumed therein. Substantially free of means less than 0.1 % by weight of the total weight and in another embodiment less than 0.01% by weight of the composition (e.g., colorant or end use).

Unless explicitly stated otherwise, all ranges described herein are meant to include all ranges subsumed therein. The term comprising is meant to encompass the terms consisting essentially of and consisting of. For the avoidance of doubt, an end use composition of this invention comprising the first and second colorant is meant to include a composition consisting essentially of the same and a composition consisting of the same. Except in the operating comparative examples, or where otherwise explicitly indicated, all numbers in this description indicating amounts or ratios of materials or conditions and/or physical properties of materials and/or use are to be understood as modified by the word "about". Mimics, as used herein means being within 12% of the hue for carmine red colorant (37 degrees) as taken with a HunterLab Labscan XE colorimeter (km, Rex) and expressed on a basis of the CIELAB color Space Scale. Color hue is defined as a hue angle and is expressed in degrees. The color hue of the colorant composition of the present invention is typically from 32.75 to 41.25. In another embodiment, the hue of the colorant composition is from 34 to 40 and yet in another embodiment from 36 to 39, including all range subsumed herein. Stable, as used herein, means having a ΔE of less than 4.5 after being stored for one (1) week at 45 °C. In another embodiment, stable means a ΔE of less than 4.0 and in still another embodiment less than 3.5 when stored at 45 °C for two (2) weeks.

### Detailed description of the invention

Regarding the first and second component suitable for use in the present invention, the same are generally classified as a salt of (sulfonatophenylazo) naphthoate and a salt of (phenylazo) napthalenedisulfonate, respectively. In one embodiment of the invention, the salt of (sulfonatophenylazo) naphthoate is a calcium salt and D&C Red No. 7 (calcium-3-hydroxy-4-[(4-methyl-2-sulfophenyl)azo]-2-naphthalenecarboxylate) whereby the salt of (phenylazo) naphthalenedisulfonate is a sodium salt and D&C Red No. 33 (disodium-5-amino-4-hydroxy-3-(phenylazo)-naphthalene-2,7-disulfonate).

Such components are often lakes and provided on a substrate such as aluminium oxide (Al₂O₃), silicon dioxide (SiO₂), aluminium silicate (Al₂SiO₅) or mixtures thereof. Typically (whether spherical particle or plate) the colorant components of the present invention are from 75 to 95% by weight substrate based on total weight of particle and including all ranges subsumed therein. In another embodiment, from 80 to 90% and still in another embodiment from 85 to 90% by weight of particle is substrate, based on total weight of the particle and including all ranges subsumed therein. Substrate typically makes up the internal portions or core of the particle or plate, as the case may be.

It is also within the scope of the invention to include optional colorant modifiers with the colorant components of the present invention. Typically, such optional colorant modifiers are provided separately or as part of the substrate and they include titanium dioxide, iron oxide (red, yellow and/or black), chromium dioxide, bismuth oxychloride, ultramarine, zinc oxide, mica or the like. When employed, such additional colorant modifier makes up from 0.01 to 25% by weight of the colorant component (each independently), based on total weight of colorant composition and including all ranges subsumed therein.

The weight ratio of first component to second component is 5:1 to 3:1, including all ratios subsumed therein. In another embodiment, the particle size of the color component, when spherical, is from 0.6 to 7 microns and in still another embodiment from 1 to 5 microns, including all ranges subsumed therein.

In yet another embodiment, when a plate, the length and width of each plate is, independent of each other, 2 to 45 microns and in yet another embodiment 3 to 40 microns, including all ranges subsumed therein. In still another embodiment the thickness of the plates can be from 75 to 650 nanometers and in even another embodiment 90 to 550 nanometers, including all ranges subsumed therein.

With respect to the end use composition, the same typically has 0.02 to 35% by weight of colorant composition and in another embodiment 0.05 to 15% by weight colorant composition, based on total weight of the end use composition and including all ranges subsumed therein. In still another embodiment, colorant composition makes up from 0.1 to 9% by weight of the total weight of the end use composition, including all ranges subsumed therein. In even another embodiment, the end use composition of the present invention comprises from 0.1 to 5% by weight colorant composition, based on total weight of end use composition and including all ranges subsumed therein.

Red 7 and Red 33, when used in the present invention, may be purchased commercially from suppliers like Neelikon, The Good Scents Company, Sensient, BASF, EMD Milipore and DeWolf Chemical. Colorant compositions, consisting essentially of Red 7 and Red 33 are often desired. In another embodiment, the colorant compositions of this invention consist of Red 7 and Red 33 supplied by Sensient.

As to the end use compositions, they include emulsions, and the same can be oil-in-water, water-in-oil or double emulsions. Such compositions may also be gels, suspensions, anhydrous or semi-anhydrous compositions.

Illustrative examples of the oils suitable for use in the end use compositions which have the colorant composition of this invention include silicone oils.

Silicone oils may be divided into the volatile and non-volatile variety. The term "volatile" as used herein refers to those materials which have a measurable vapor pressure at ambient temperature. Volatile silicone oils are preferably chosen from cyclic or linear polydimethylsiloxanes containing from 3 to 9, and preferably, from 4 to 5 silicon atoms. Nonvolatile silicone oils useful in this invention include polyalkyl siloxanes, polyalkylaryl siloxanes and polyether siloxane copolymers. Such essentially non-volatile polyalkyl siloxanes useful herein include, for example, polydimethylsiloxanes (like dimethicone) with viscosities of from 5 to 100,000 centistokes at 25°C.

An often-preferred silicone source is a cyclopentasiloxane and dimethiconol solution.

Suitable esters for use to make end use compositions in this invention include:
(1) Alkenyl or alkyl esters of fatty acids having 10 to 20 carbon atoms like isopropyl palmitate, isopropyl isostearate, isononyl isonanonoate, oleyl myristate, isopropyl myristate, oleyl stearate, and oleyl oleate;
(2) Ether-esters such as fatty acid esters of ethoxylated fatty alcohols;
(3) Polyhydric alcohol esters such as ethylene glycol mono- and di-fatty acid esters, diethylene glycol mono- and di-fatty acid esters, polyethylene glycol (200-6000) monoand di-fatty acid esters, propylene glycol mono- and di-fatty acid esters, polypropylene glycol 2000 monooleate, polypropylene glycol 2000 mono stearate, ethoxylated propylene glycol monostearate, glyceryl mono- and di-fatty acid esters, polyglycerol polyfatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxy-ethylene sorbitan fatty acid esters;
(4) Wax esters such as beeswax, spermaceti, myristyl myristate, stearyl stearate, tribehenin wax (and other waxes with a melting point of over 20°C); and
(5) Sterol esters, of which soya sterol and cholesterol fatty acid esters are examples thereof.

Still other oils that may be used in end use compositions of this invention include soybean oil, sunflower oil, coconut oil, palm kernel oil, castor oil, rapeseed oil, palm oil, grape seed oil, shea butter, cocoa butter, caprylic/capric triglyceride, safflower oil, fish oil, mixtures thereof as well as sugar esters of fatty acids such as sucrose polybehenate and sucrose polycottonseedate.

Even other oils suitable for use include mineral oil, jajoba oil, isoparaffins, C₁₂-C₁₅ alkyl benzoates, polyalphaolefins, isohexadecane, siliconized waxes like siliconized beeswax, vegetable wax, petrolatum, mixtures thereof (including with those oils above) or the like. Soybean and sunflower oil are often preferred triglyceride oils as are any of those typically classified as an emollient.

Caprylic capric triglyceride is another oil often preferred oil for use in the end use compositions of the present invention.

Adjusters suitable to modify the pH of the emulsions may be used. Such pH adjusters include Mg(OH)₂, NaOH, KOH, CaCO₃, H₂SO₄, HCl, C₆ H₈ O₇ (i.e., citric acid), triethanolamine or mixtures thereof. The pH adjusters are added at amounts such that the pH of the resulting end use composition is from 4.5 to 7.5 including all ranges subsumed therein. Chelators, like EDTA, may optionally be used, typically from 0.01 to 2.0% by weight is used (when desired) based on total weight of the end use composition. Liquid oligomers like polyisobutylene may also be used at amounts of 0.4 to 1.5% by weight.

The pH of end use compositions may be assessed by using conventional instrumentation such as a pH meter made commercially available from Thermo Scientific^{®}.

When emulsions are desired, as the end use composition, the emulsifiers suitable for use in this invention typically have an HLB from 2.5 to 10.5, and preferably, from 3 to 9.5, and most preferably, from 3 to 8.5, including all ranges subsumed therein.

Illustrative examples of the emulsifiers suitable for use in this invention are propylene glycol isostearate, glycol stearate sorbitan sesquioleate, lecithin, oleth-2, stearth-2, ceteth-2 glyceryl stearate, PEG-30 dipolyhydroxystearate, laureth-4r, PEG-8 dioleate, sorbitan laurate and PEG-7 glyceryl cocoate.

Still other emulsifiers suitable for use include glycol distearate, glyceryl oleate, sorbitan monooleate, sorbitan tristearate, sorbitan trioleate, sorbitan monopalmitate, lauryl PEG-10, (trimethylsiloxy)silylethyl dimethicone (Dow Corning^{®} ES-5300) or mixtures thereof.

Emulsifiers typically make up from 2.5 to 10, and preferably, from 3.5 to 8, and most preferably, from 4.5 to 7.5% by weight of the emulsion (i.e., end use composition) including all ranges subsumed therein. Those with an HLB over 8 are typically preferred for emulsions that are water continuous. It is also within the scope of the invention for the emulsions to be pickering emulsions.

Lipsticks and balms suitable for use with the composition of the present invention can include a hydrophobic organic solid of melting point in excess of 20°C, and which assists in forming a solid structure for the cosmetic stick. Of particular utility are waxes. The waxes are low-melting organic compounds or mixtures of high molecular weight substances, are solid at room temperature and are generally similar in composition to fats and oils, except that they essentially contain no glycerides. Some are hydrocarbons; others are esters of fatty acids and alcohols. Waxes are thermoplastic, but since they are not high polymers, they are not considered in the family of plastics. Natural, mineral and synthetic waxes may all be employed. Among the natural waxes are those of animal origin (e.g. beeswax, lanolin, shellac wax), vegetable (carnauba, candelilla, bayberry, sugarcane wax) and mineral (ozokerite, ceresin, montan, paraffin, microcrystalline petroleum and petrolatum wax). Snythetic waxes include polyol ether-esters such as "carbowax" and hydrocarbon-type waxes.

Typically, the amount of active agent employed in the end use composition comprising colorant composition of the present invention is from 0.05 to 10%, and preferably, from 1 to 6%, and most preferably, from 2 to 5% by weight, based on total weight of the end use composition and including all ranges subsumed therein.

When desired, it is within the scope of the present invention to optionally include in the end use compositions with the colorant composition of the present invention an oil soluble active in the oil phase of such end use composition. The only limitation with respect to such optional oil soluble active agent is that the same is suitable to provide a skin benefit when topically applied.

Illustrative examples of the types of oil soluble actives (or benefit agents) that may optionally be used in this invention include vitamins like Vitamin A, D, E and K (and their oil soluble derivatives), sunscreens like ethylhexylmethoxycinnamate, bis-ethyl hexyloxyphenol methoxyphenol triazine, 2-ethylhexyl-2-cyano-3,3-diphenyl-2-propanoic acid, drometrizole trisiloxane, 3,3,5-trimethyl cyclohexyl 2-hydroxybenzoate, 2-ethylhexyl-2-hydroxybenzoate or mixtures thereof.

Other optional oil soluble actives suitable for use include resorcinols like 4-hexyl resorcinol, 4-phenylethyl resorcinol, 4-cyclopentyl resorcinol, 4-cyclohexyl resorcinol 4-isopropyl resorcinol or mixture thereof. Also, 5-substituted resorcinols like 4-cyclohexyl-5-methylbenzene-1,3-diol, 4-isopropyl-5-methylbenzene-1,3-diol, mixtures thereof or the like may be used. The 5-substituted resorcinols, and their synthesis are described in commonly assigned U.S. Published Patent Application No. 2016/0000669A1.

Even other optional oil soluble actives suitable for use include omega-3 fatty acids, omega-6 fatty acids, climbazole, farnesol, ursolic acid, myristic acid, geranyl geraniol, oleyl betaine, cocoyl hydroxyethyl imidazoline, hexanoyl sphingosine, 12-hydroxystearic acid, petroselinic acid, conjugated linoleic acid, terpineol, thymol, mixtures thereof or the like.

When the optional (i.e., 0.0% by weight) oil soluble active agent is used in the oil phase of the end use composition, with the colorant composition of this invention, it typically makes up from 0.001 to 8%, and preferably, from 0.05 to 4.5%, and most preferably, from 0.1 to 3% by weight of the end use composition, based on total weight of the end use composition, the colorant composition of the invention, and including all ranges subsumed therein.

Preservatives can desirably be incorporated into the end use compositions of this invention (i.e., those with water) to protect against the growth of potentially harmful microorganisms, although it is within the scope of the invention for the such end use compositions to be preservative free. Suitable traditional preservatives for use in this invention, without limitations, are alkyl esters of para-hydroxybenzoic acid. Other preservatives include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are iodopropynyl butyl carbamate (IPBC), phenoxyethanol, 1,2-octanediol, hydroxyacetophenone, ethylhexylglycerine, hexylene glycol, methyl paraben, propyl paraben, imidazolidinyl urea, sodium dehydroacetate, and propanediol. The preservatives should be selected having regard for the use of the composition and possible incompatibilities between the preservatives and other ingredients in the emulsion. Preservatives are preferably employed in amounts ranging from 0.01% to 2% by weight of the total weight of the emulsion or end use composition, including all ranges subsumed therein. Combinations of 1,2-octanediol and phenoxyethanol, or iodopropynyl butyl carbamate and phenoxyethanol are preferred, with phenoxyethanol and 1,2-octanediol, collectively and preferably, making up less than 1.8.% by weight of the total weight of the end use composition. Also preferred is a preservative system with hydroxyacetophenone alone or in a mixture with other preservatives.

Thickening agents are suitable for use in the end use compositions of the present invention. Particularly useful are the polysaccharides. Examples include fibers, starches, natural/synthetic gums and cellulosics. Representative of the starches are chemically modified starches such as sodium hydroxypropyl starch phosphate and aluminum starch octenylsuccinate. Tapioca starch is often preferred, as is maltodextrin. Suitable gums include xanthan, sclerotium, pectin, karaya, arabic, agar, guar (including Acacia senegal guar), carrageenan, alginate and combinations thereof. Suitable cellulosics include hydroxypropyl cellulose, hydroxypropyl methylcellulose, ethylcellulose, sodium carboxy methylcellulose (cellulose gum/carboxymethyl cellulose) and cellulose (e.g. cellulose microfibrils, cellulose nanocrystals or microcrystalline cellulose). Sources of cellulose microfibrils include secondary cell wall materials (e.g. wood pulp, cotton), bacterial cellulose, and primary cell wall materials. Preferably the source of primary cell wall material is selected from parenchymal tissue from fruits, roots, bulbs, tubers, seeds, leaves and combination thereof; more preferably is selected from citrus fruit, tomato fruit, peach fruit, pumpkin fruit, kiwi fruit, apple fruit, mango fruit, sugar beet, beet root, turnip, parsnip, maize, oat, wheat, peas and combinations thereof; and even more preferably is selected from citrus fruit, tomato fruit and combinations thereof. A most preferred source of primary cell wall material is parenchymal tissue from citrus fruit. Citrus fibers, such as those made available by Herbacel^{®} as AQ Plus can also be used as source for cellulose microfibrils. The cellulose sources can be surface modified by any of the known methods including those described in Colloidal Polymer Science, Kalia et al., "Nanofibrillated cellulose: surface modification and potential applications" (2014), Vol 292, Pages 5-31.

Synthetic polymers are yet another class of effective thickening agent. This category includes crosslinked polyacrylates such as the Carbomers, polyacrylamides such as Sepigel 305 and taurate copolymers such as Simulgel EG and Aristoflex AVC, the copolymers being identified by respective INCI nomenclature as Sodium Acrylate/Sodium Acryloyldimethyl Taurate and Acryloyl Dimethyltaurate/Vinyl Pyrrolidone Copolymer. Another preferred synthetic polymer suitable for thickening is an acrylate-based polymer made commercially available by Seppic and sold under the name Simulgel INS100. Calcium carbonate, fumed silica, and magnesium-aluminum-silicate may also be used. Others classified as thickening fillers may be used, such as talc, kaolin and bentonite. Binding agents that thicken like zinc stearate and sodium stearate may also be used.

Amounts of the thickening agent, when used, may range from 0.001 to 22%, and preferably, from 0.1 to 17%, and most preferably, from 0.2 to 16% by weight of the end use composition, based on total weight of the end use composition and including all ranges subsumed. Maltodextrin, xanthan gum, and carboxymethyl cellulose are often desired.

When the end use composition is a powder, however, zinc stearate, talc and zinc oxide may be used, with talc making up from 65 to 80% by weight and zinc oxide making up from 20 to 30% by weight of the end use composition (i.e, powder). In powders, zinc stearate is often present from 0.5 to 4.5% by weight of the total weight of the powder.

Fragrances, fixatives and exfoliants may optionally be included in end use compositions with the colorant composition of the present invention. Each of these substances may range from about 0.02 to about 5%, preferably between 0.1 and 3% by weight. Antioxidants (from 0 to 10% by weight) may be used if desired. Illustrative examples include vitamin C, and especially Tinogard TT, Tinogard S and Tinogard DA made available by BASF.

Conventional humectants may optionally be employed with the colorant compositions of the present invention to assist in moisturizing skin when such end use compositions are topically applied. These are generally polyhydric alcohol type materials. Typical polyhydric alcohols include glycerol (i.e., glycerine or glycerin), propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. Most preferred is glycerin, propylene glycol or a mixture thereof. The amount of humectant employed may range anywhere from 0.0 to 10 to 15% by weight of the total weight of the end use composition.

The end use compositions with colorant composition of the present invention may optionally include water-soluble active agents like Vitamin B₂, Vitamin B₃ (niacinamide), Vitamin B₆, Vitamin C and the like. Water-soluble derivatives of the vitamins may also be employed. For instance, Vitamin C derivatives such as ascorbyl tetraisopalmitate, magnesium ascorbyl phosphate and ascorbyl glycoside may be used. Other optional water-soluble actives suitable for use in the water phase of the emulsions include extracts like sage, aloe vera, green tea, grapeseed, thyme, chamomile, liquorice or rosemary extract or mixtures thereof. Still other water-soluble actives suitable for use include alpha hydroxyacids like lactic and glycolic acid, beta hydroxy acids like salicylic acid, amino acids like arginine, lysine, glutamine, glycine, glutamic acid (and its derivatives, like pyroglutamic acid), alanine, valine and agents like ferulic acid, hyaluronic acid, and allantoin. Water-soluble sunscreens like ensulizole may also be used. Total amount of optional water-soluble active agents (including mixtures) when present in the end use compositions with the colorant composition of the present invention may range from 0.0 to 15%, preferably from 0.001 to 10%, optimally from 0.01 to 4% by weight, based on total weight of the end use composition and including all ranges subsumed therein.

In the invention, the active agent is 4-ethyl resorcinol, retinyl propionate or a mixture thereof.

In another embodiment of the invention, the colorant composition and end use composition are substantially free of carmine red. In still another embodiment of the invention, the end use composition is free of (0.0% by weight) of carmine red.

When not anhydrous or substantially anhydrous, water makes up from 25 to 95%, in another embodiment, 35 to 85% and still in another embodiment, 40 to 79% by weight of the end use composition, based on total weight of end use composition an including all ranges subsumed therein.

When making the end use compositions with the colorant composition of the present invention, the desired ingredients may be mixed to produce water and oil phases. The same may be mixed under moderate shear with emulsifier under atmospheric conditions with temperature being from ambient to 85°C. Mixing may be accomplished in a commercially available mixer like a rotor/stator high shear mixer made commercially available by Silverson or Charles Ross & Son. Typically, shear rate can vary and is preferable set such that the resulting emulsions are not aerated to the point of displaying visual air pockets. Often, mixing is accomplished with rotation being set from 100 to 1,500, and preferable, from 200 to 1,200, and most preferably from 400 to 1000 rpm, including all ranges subsumed therein. Use of a homogenizing system such as a Sonic Corporation Sonolator^{™} may also be used to make macroemulsion, with pressure set from 100 to 400, and preferably, from 250 to 650 psi. Moderate shear and heat may be used, under atmospheric conditions, to prepare anhydrous and substantially anhydrous end use compositions. Conventional pressure applications may be employed when making anhydrous and substantially anhydrous end use product.

The packaging for the end use compositions with the colorant composition of this invention is typically a bottle, pencil applicator, cosmetic puff, tube or jar. Other suitable packages include blister pack or sachets.

The products with the colorant composition of the present invention may also be dispensed from automatic dispensers or packaging pressurized with propellant.

The Examples provided are to facilitate an understanding of the invention. They are not intended to limit the scope of the claims.

### Example 1

The following ingredients were mixed under moderate shear and ambient temperature to produce the resulting end use gel composition.

| **Gel Composition** | **Weight percent** |
|---|---|
| **Ingredient Name** | **%** |
| Propylene glycol | 2.0 |
| Disodium Ethylene diamine tetraacetic acid | 0.04 |
| Phenoxyethanol | 0.20 |
| Triethanolamine | 0.10 |
| Glycerin | 1.0 |
| Preservative | 0.20 |
| Water | Balance |
| Ammonium acryloyl dimethyl taurate/VP copolymer | 2.0 |
| Total | 100 |

### Example 2

Color Stability of Carmine Replacement consistent with this invention* (4:1 weight ratio) with Ethyl Resorcinol (ER) and Retinyl Propionate (RP) in the Gel Composition of Example 1, water to balance

| **Description** | **ΔE Week 1 at 45°C** | **ΔE Week 2 at 45°C** |
|---|---|---|
| Gel, 0.5% carmine+0.25% ER | 9.13 | 10.17 |
| Gel, 0.5% carmine+0.3% RP | 5.10 | 4.31 |
| Gel, 0.25% 4:1 Red 7:Red 33 +0.25% ER | 1.13 | 2.96 |
| Gel, 0.25% 4:1 Red 7:Red 33 +0.3% RP | 2.96 | 2.36 |
| Gel, 0.5% Red 30+0.25% ER | 2.69 | 9.33 |
| Gel, 0.5% Red 30+0.3% RP | 4.79 | 3.12 |
| Gel, 0.5% Red 40+0.25% ER | 4.50 | 28.19 |
| Gel, 0.5% Red 40+0.3% RP | 2.95 | 5.52 |

| | | |
|---|---|---|
| *Red 7 and Red 33 supplied by Sensient | | |

The gel compositions described in this example were stored at 45°C and color change was assessed with a Hunterlab Labscan XE Colorimeter. Unexpectedly, when including colorant composition consistent with the present invention in end use gel composition, the color of the gel compositions was stable when active (oil and water soluble) was used with the colorant composition of the present invention.

### Example 3

An anhydrous rouge composition may be made by combining the ingredients below.

| **INGREDIENT** | **% By Weight** |
|---|---|
| Red 7:Red 33, 4:1 weight ratio | 24.5 |
| Talc | balance |
| Kaolin | 3.5 |
| Zinc Stearate | 3.5 |
| Mica | 25.0 |
| Mica (and) Titanium Dioxide (and) Silica) (33:33:33) | 4.5 |
| Yellow Iron Oxide (and) Titanium Dioxide (50:50) | 22.0 |
| Emollient | 9.5 |
| Antioxidant | 0.5 |
| Chromium dioxide | 0.5 |

### Example 4

A powder composition may be made by combining the ingredients below. Conventional pressure applications may be applied to produce the desired powder.

| **Ingredients** | **%** |
|---|---|
| Talc | balance |
| Zinc oxide | 24.0 |
| Zinc stearate | 2.5 |
| Yellow Iron Oxide | 0.3 |
| Red 7: Red 33, 3:1 weight ratio | 1.0 |

### Example 5

A semi-anhydrous cosmetic lipstick may be made by combining the ingredients below.

| **Ingredient** | **% Weight** |
|---|---|
| Castor oil | 19.5 |
| Isopropyl palmitate | 11.6 |
| Caprylic/capric triglyceride | 12.8 |
| Lanolin | 9.5 |
| Red 7: Red 33, 12:1 weight ratio | 7.0 |
| Candelilla wax | 6.6 |
| Propylene glycol myristyl ether acetate | 6.0 |
| Glycerol | 5.0 |
| Water | Balance |
| Titanium dioxide | 4.7 |
| Beeswax | 7.6 |
| Ozokerite wax | 2.5 |
| lecithin | 1.0 |
| Polybutylene | 0.8 |
| Carnauba wax | 0.4 |

## Claims

1. A composition comprising a colorant composition comprising:
(a) a first component comprising a salt of (sulfonatophenylazo) naphthoate;
(b) a second component comprising a salt of (phenylazo) naphthalenedisulfonate, the first and second components in a weight ratio of 5:1 to 3:1, the colorant composition mimics the color of cochineal red and is stable for at least one week when stored at 45°C; wherein the composition comprises an active, wherein the active is 4-ethyl resorcinol, retinyl propionate or a mixture thereof.

2. The composition according to claim 1 wherein the first component consists of (sulfonatophenylazo) naphthoate and the second component consists of (phenylazo) naphthalenedisulfonate.

3. The composition according to claim 1 wherein the first and second components are particles, plates or both, the particles being from 0.5 to 7 microns, preferably 0.6 to 7 microns, more preferably 1 to 5 microns, and the plates having a length and width, independently, from 1 to 50 microns, preferably 2 to 45 microns, more preferably 3 to 40 microns and a thickness from 75 to 650 nanometers, preferably 90 to 550 nanometers.

4. The composition according to any preceding claim wherein the first component is calcium-3-hydrox-4-[(4-methyl-2-sulfophenyl)azo]-2-naphthalenecarboxylate and the second component is disodium-5-amino-4-hydroxy-3-(phenylazo)-naphthalene-2,7-disulfonate

5. The composition according to claim 1 wherein the composition comprises from 0.02 to 35, preferably 0.05 to 15, more preferably 0.1 to 9% by weight of the colorant composition

6. The composition according to claim 1 wherein the composition is free of carmine red.

## Patentansprüche

1. Zusammensetzung, umfassend eine Farbmittelzusammensetzung, umfassend:
(a) eine erste Komponente, umfassend ein Salz von (Sulfonatophenylazo)naphthoat;
(b) eine zweite Komponente, umfassend ein Salz von (Phenylazo)naphthalindisulfonat,
wobei die erste und die zweite Komponente in einem Gewichtsverhältnis von 5:1 bis 3:1 vorliegen, wobei die Farbmittelzusammensetzung die Farbe von Cochenillerot imitiert und bei einer Lagerung bei 45°C mindestens eine Woche lang stabil ist, wobei die Zusammensetzung einen Wirkstoff umfasst, wobei der Wirkstoff
4-Ethyl-resorcin-retinylpropionat oder eine Mischung davon darstellt.

2. Zusammensetzung nach Anspruch 1, wobei die erste Komponente aus (Sulfonatophenylazo)naphthoat besteht und die zweite Komponente aus (Phenylazo)naphthalindisulfonat besteht.

3. Zusammensetzung nach Anspruch 1, wobei die ersten und zweiten Komponenten Partikel, Platten oder beides darstellen, wobei die Partikel eine Größe von 0,5 bis 7 Mikrometer, vorzugsweise von 0,6 bis 7 Mikrometer, bevorzugter 1 bis 5 Mikrometer aufweisen und die Platten eine Länge und Breite, unabhängig voneinander, von 1 bis 50 Mikrometer, vorzugsweise 2 bis 45 Mikrometer, bevorzugter von 3 bis 40 Mikrometer, und eine Dicke von 75 bis 650 Nanometer, vorzugsweise von 90 bis 550 Nanometer, aufweisen.

4. Zusammensetzung nach einem vorhergehenden Anspruch, wobei die erste Komponente Calcium-3-hydroxy-4-[(4-methyl-2-sulfophenyl)azo]-2-naphthalincarboxylat und die zweite Komponente Dinatrium-5-amino-4-hydroxy-3-(phenylazo)-naphthalin-2,7-disulfonat ist.

5. Zusammensetzung nach Anspruch 1, wobei die Zusammensetzung 0,02 bis 35 Gewichts-%, vorzugsweise 0,05 bis 15 Gewichts-%, bevorzugter 0,1 bis 9 Gewichts-% der Farbmittelzusammensetzung enthält.

6. Zusammensetzung nach Anspruch 1, wobei die Zusammensetzung frei von Karminrot ist.

## Revendications

1. Composition comprenant une composition de colorant comprenant :
(a) un premier constituant comprenant un sel de (sulfonatophénylazo)naphtoate ;
(b) un second constituant comprenant un sel de (phénylazo)-naphthalènedisulfonate,
les premier et second constituants dans un rapport de masse de 5:1 à 3:1, la composition de colorant reproduit la couleur de rouge cochenille et est stable pendant au moins une semaine lorsque stockée à 45°C ; dans laquelle la composition comprend un actif, dans laquelle l'actif est le 4-éthylrésorcinol, propionate de rétinyle ou un mélange de ceux-ci.

2. Composition selon la revendication 1, dans laquelle le premier constituant consiste en (sulfonatophénylazo)naphthoate et le second constituant consiste en (phénylazo)naphthalènedisulfonate.

3. Composition selon la revendication 1, dans laquelle les premier et second constituants sont des particules, plaques, ou les deux, les particules étant de 0,5 à 7 microns, de préférence 0,6 à 7 microns, encore mieux 1 à 5 microns, et les plaques ayant une longueur et une largeur, indépendamment, de 1 à 50 microns, de préférence 2 à 45 microns, encore mieux 3 à 40 microns et une épaisseur de 75 à 650 nanomètres, de préférence 90 à 550 nanomètres.

4. Composition selon l'une quelconque des revendications précédentes, dans laquelle le premier constituant est le calcium-3-hydroxy-4-[(4-méthyl-2-sulfophényl)azo]-2-naphthalènecarboxylate et le second constituant est le disodium-5-amino-4-hydroxy-3-(phénylazo)-naphthalène-2,7-disulfonate.

5. Composition selon la revendication 1, dans laquelle la composition comprend de 0,02 à 35, de préférence 0,05 à 15, encore mieux 0,1 à 9 % en masse de la composition de colorant.

6. Composition selon la revendication 1, dans laquelle la composition est exempte de rouge carmin.
